# EUROPEAN PATENT SPECIFICATION

(11) **EP 0 380 924 B1**
(45) Date of publication and mention of the grant of the patent: **22.06.1994**
(21) Application number: 90100314.5
(22) Date of filing: 08.01.1990
(51) Int. Cl.: C07C 217/48

(54) **Process for the preparation of fluoxetine hydrochloride**
Verfahren zur Herstellung von Fluoxetinhydrochlorid
Procédé pour la préparation du chlorohydrate de fluoxétine

(30) Priority: 10.01.1989 IT 1904989
(43) Date of publication of application: 08.08.1990
(73) Proprietor: Magnone, Grato, Milan (IT)
(72) Inventor: Magnone, Grato, Milan (IT)
(74) Representative: Gervasi, Gemma, Dr.

(56) References cited:
- GB-A- 2 060 618
- US-A- 4 314 081
- THE JOURNAL OF ORGANIC CHEMISTRY, vol. 53, no. 13. 24th June 1988, pages 2916-2920, American Chemical Society, US; M. SREBNIK et al.: "Chiral synthesis via organoboranes. 18. Selective reductions. 43. Diisopinocampheylchloroborane as an excellent chiral reducing reagent for the synthesis of halo alcohols of high enantiomeric purity. A highly enantioselective synthesis of both optical isomers of tomoxetine, fluoxetine, and nisoxetine"
- THE JOURNAL OF ORGANIC CHEMISTRY, vol. 53, no. 17, 19th August 1988, pages 4081-4085, American Chemical Society, US; Y. GAO et al.: "Asymmetric synthesis of both enantiomers of tomoxetine and fluoxetine. Selective reduction of 2,3-epoxycinnamyl alcohol with red-A1"
- JOURNAL OF THE AMERICAN CHEMICAL SOCIETY, vol. 109, no. 19, 16th September 1987, pages 5856-5858, American Chemical Society, US; R. NOYORI et al.: "Asymmetric hydrogenation of beta-keto carboxylic esters. A practical, purely chemical access to beta-hydroxy esters in high enantiomeric purity"

## Description

### Prior art

Fluoxetine hydrochloride is a drug which is employed on a large scale as a selective inhibitor of serotonin uptake (Wong, D.T. et al., Drug Dev. Res., 1985, 6, 397) and in the treatment of depressive syndromes (Chovinard, G.A.; Clin. J. Psychiatry, 1985, 46, 32).

The preparation of fluoxetine hydrochloride as referred to for instance in the USP 4018895, 4194009 and 4314081 of the Eli-Lilly Company.

The preparation described in those patents is carried out according to the following stages:
compound (II) 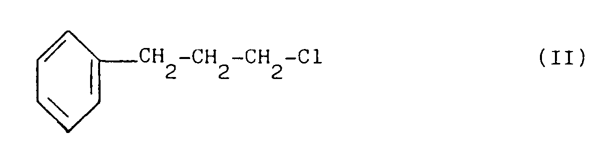
is brominated to obtain compound (III) 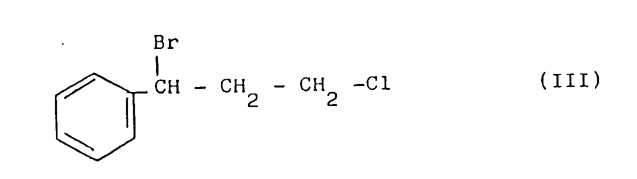
Compound (III) is treated with 4-trifluoromethylphenol leading to compound (IV) 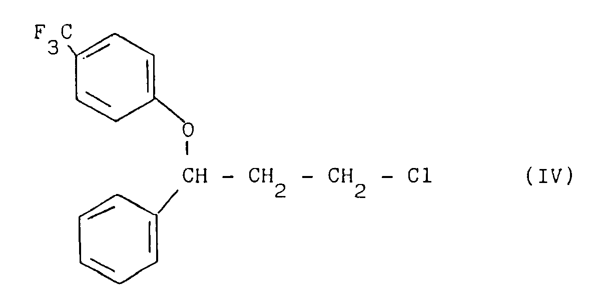
compound (IV), by substitution of Cl with methylamine yields fluoxetine (V) 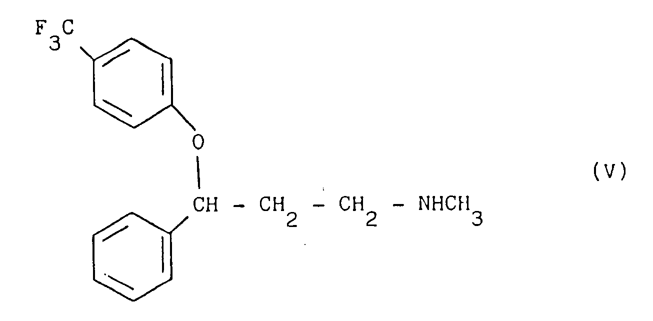
from which, by treatment with HCl, fluoxetine hydrochloride is obtained. The described reaction sequence presents various drawbacks.

In particular, the passage from compound (III) to compound (IV) gives modest yields even by long reaction times and requires the employment of sodium hydride in dimethylformamide; the passage from compound (IV) to compound (V) requires high temperature and pressure and leads to the formation of byproducts which make it difficult to crystallize the final hydrochloride.

Also the process which utilizes as an intermediate the N,N-dimethyl derivative of compound (V) presents difficulties in the commercial application. In fact the demethylation with cyanogen bromide is not practical.

A more recent patent (Spanish patent 7175 of the INKE Company) employs, in the preparation of fluoxetine hydrochloride, the use of compound (VI) as an intermediate. 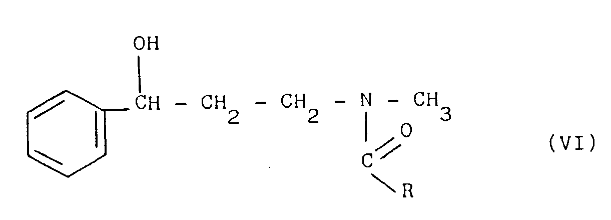
in which R is an alkyl group.

Compound (VI) is transformed into compound (VII) 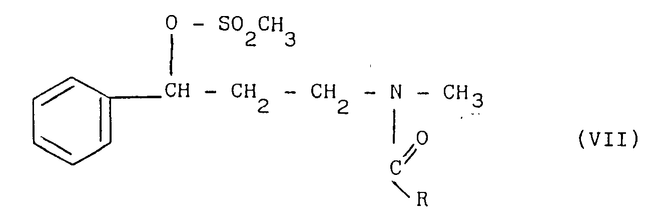
From compound (VII), by treatment with 4-CF₃C₆H₄OH, compound (VIII) is obtained 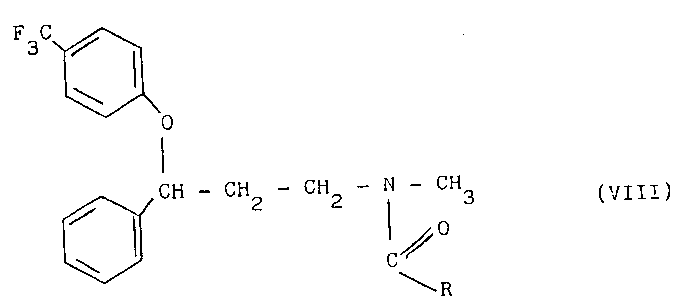
which by hydrolysis yields fluoxetine.

This process also presents various drawbacks, among which the scarce availability of compound (VI), the instability of compound (VII) and the low yield in the passage from compound (VII) to compound (VIII).

M. Srebnik et al. in J. Org. Chem., 1988, 53, 2916 - 2920 describe an enantioselective synthesis of both optical isomers of fluoxetine.

### Summary of the invention

We have now found a new process for the preparation of fluoxetine hydrochloride (I) 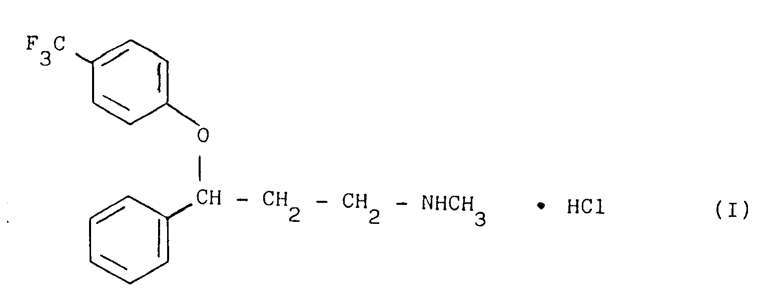
which presents considerable advantages with respect to the known processes.

Said process is characterized by the fact that:
a) ethyl benzoyl acetate (IX) is reduced with the aid of metal hydrides in a hydroalcoholic solvent, at a controlled pH to obtain ethyl 3-hydroxy-3-phenylpropionate (X);
b) compound (X) is treated with methylamine in an alcoholic solvent to obtain the N-methylamide of 3-hydroxy-3-phenylpropionic acid (XI);
c) compound (XI) is treated with 4-trifluoromethylphenol and optionally with activating substances in an organic solvent to obtain 3-phenyl-3-[4-(trifluoromethyl)phenoxy]-N-methylpropanamide (XII);
d) compound (XII) is reduced with the aid of a metallic hydride in an organic solvent to obtain fluoxetine (V) from which, by treatment with HCl, fluoxetine hydrochloride is obtained (I).

This process can be easily applied on a commercial scale with a very high overall yield, and it has furthermore the advantage of utilising as a starting compound a product which is easily available and very inexpensive.

### Detailed description of the invention

The characteristics and the advantages of the process for the preparation of fluoxetine hydrochloride (I) according to the present invention, will be better illustrated in the following detailed description.

Said process is carried out through the following steps:
a) ethyl benzoyl acetate (IX) 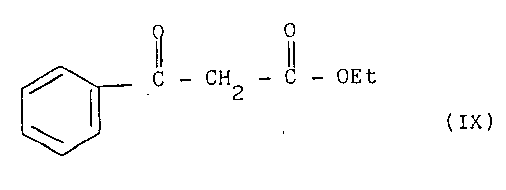 is reduced with the aid of metal hydrides, yielding compound (X) 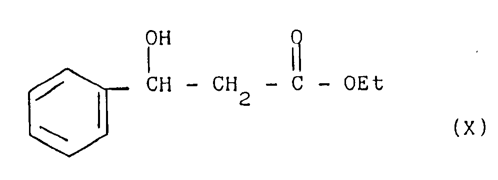
b) compound (X) is treated with methylamine to obtain compound (XI) 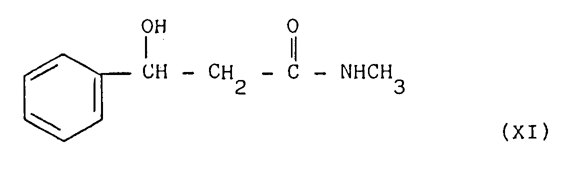
c) compound (XI) is treated with 4-trifluoromethylphenol to obtain compound (XII) 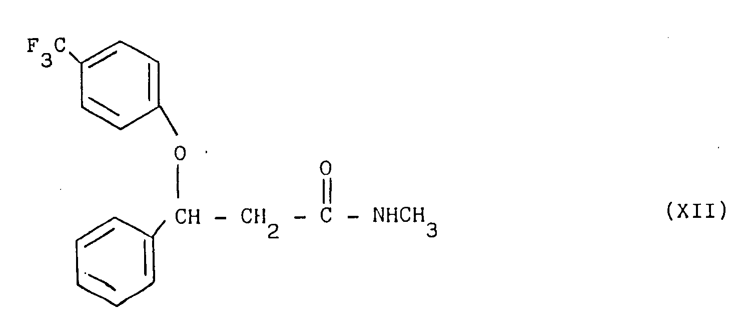
d) compound (XII) is reduced to obtain compound (V) 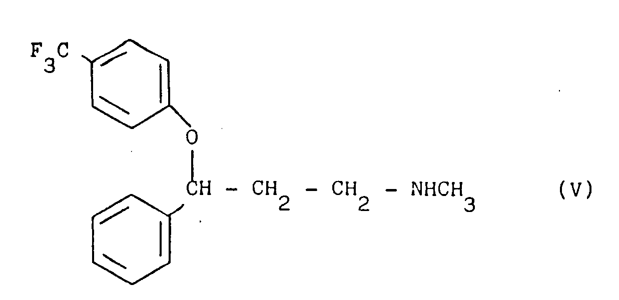 from which, by treatment with HCl, fluoxetine hydrochloride (I) is obtained.

Stage a) is carried out in a hydroalcoholic solvent by treatment with metal hydrides at a slightly acid pH.

As a solvent a mixture of methanol and water in a ratio between 4:1 to 5:1 is preferably employed; as metal hydride, NaBH₄ in 1N NaOH is employed, in an amount between a molar equivalent and 10% excess with respect to (IX). The reaction is carried out at a temperature between 10 and 15°C, while the pH is kept between 6 and 7 by addition of 50% acetic acid, under stirring. The product is recovered by partial evaporation of the solvent under reduced pressure, dilution with water, solvent extraction, for example with methylene chloride, drying and evaporation of the organic phase.

In a reaction time between 1 and 3 hours a yield of between 80 and 95% is obtained.

Stage b) is carried out by treating a solution of compound (X) in methanol (one mole in 200-300 ml) with a 35% by weight solution of methylamine in methanol at room temperature under stirring. The reaction is completed in 24 hours.

Product (XI) is recovered by evaporation to dryness and is employed without further purification in step c).

Step c) is carried out in anhydrous tetrahydrofuran with a stoichiometric amount of the reagents, at a temperature between 15 and 20°C, under stirring.

In this stage activating substances are suitably added, such as methanesulfonic acid, 4-toluenesulfonic acid, trifluoromethanesulfonic acid, or triphenylphosphine and diethyl azodicarboxylate.

The product is recovered by evaporation to dryness, addition of boiling hexane, separation of extraneous products and concentration.

The yield is approximately 90%.

Step d) is carried out by treating compound (XII) with metal hydrides such as diborane or LiAlH₄ in anhydrous tetrahydrofuran.

Preferably the treatment is carried out by feeding slowly a solution of compound (XII) in THF under stirring in a nitrogen atmosphere, to a boiling mixture of LiAlH₄ in THF and keeping 4 hours at the boiling point, under reflux.

The molar ratio between LiAlH₄ and (XII) is comprised between 0.7 and 0.9.

The reaction is completed after four hours.

The reaction mixture is cooled, a mixture of acetone and ethyl acetate is added in order to destroy the excess of hydride, the solution is diluted with NaOH, extracted twice with hexane/ethyl acetate 2:1, the extract is washed with water and brought to dryness, obtaining compound (V).

Compound (V) is taken up with a small amount of ether and treated with gaseous HCl; after cooling to 3-5 °C for 12 hours a precipitate of fluoxetine hydrochloride (I) is obtained with a yield of 70-80%.

In order to illustrate the process of the present invention, without limiting it, the following example of preparation of fluoxetine hydrochloride is reported.

### EXAMPLE 1

### a) Ethyl 3-hydroxy-3-phenylpropionate (X)

One mole of ethyl benzoyl acetate in 700 ml methanol and 150 ml water is treated with a molar equivalent of NaBH₄ dissolved in 1N NaOH, which is added dropwise at 10-15°C, under stirring, keeping the pH between 6 and 7 by addition of 50% acetic acid.

Once the reaction is terminated (which is followed in its course by TLC), part of the solvent is evaporated under reduced pressure; after dilution with water the solution is extracted with methylene chloride. The organic phase is evaporated to dryness, obtaining product (X) with a yield of 90%.

¹H NMR: 90 MHz (CDCl₃) δ 7.4(5H), 5.15(1H), 4.2(2H), 3.3(1H), 2.7 (2H), 1.25 (3H)

### b) 3-hydroxy-3-phenylpropionic acid N-methylamide (XI)

The ester (X) obtained in the preceeding step (0.9 moles) is dissolved in 250 ml methanol and treated at room temperature with 100 ml of 35% methylamine. After 16 hours, further 80 ml methylamine are added. The development of the reaction is followed by TLC, and after 24 hours the reaction is completed.

By evaporation to dryness a quantitative yield of product (XI) is obtained, which can be used without further purification in the successive step.

¹H NMR: 90 MHz (CDCl₃) δ 7.35 (5H), 6.1 (1H), 5.1 (1H), 4.3 (1H), 4.3 (1H) 2.85 (3H), 2.5 (2H).

### c) 3-phenyl-3-[4-trifluoromethyl)phenoxy]-N-methylpropanamide (XII)

Compound (XI) dissolved in 50 ml anhydrous THF is treated with 20 mmoles of 4-trifluoromethylphenol and, thereafter, with 20 mmoles triphenylphosphine and 20 mmoles diethyl azodicarboxylate, under stirring and keeping the temperature between 15 and 20 °C. After a few hours, the reaction, followed by TLC, is completed. The solution is evaporated to dryness and taken up with boiling hexane. By cooling, triphenyl phosphine as oxide and the ethyl ester of hydrazinodicarboxylic acid are separated.

One may repeat the operation and at the end, by concentration, amide (XII) with a yield of 90% is isolated.

^{1N} NMR: 90 MHz (CDCl₃) δ 6.8-7.8 (9H), 5.75 (1H), 2.6-3 (5H)

### d) Fluoxetine hydrochloride (I)

Amide (XII) obtained in the preceeding step, 0,18 mmoles, in 60 ml THF is added dropwise under stirring and in the presence of nitrogen to a boiling mixture of 0.15 mmoles LiAlH₄ in 50 ml THF. After 4 hours under reflux, the reaction is completed.

After cooling, a mixture of acetone and ethyl acetate is added to destroy the excess of hydride, then the solution is diluted with 10% NaOH. The mixture is extracted twice with hexane/ethyl acetate 2:1, washed with water, and dried. The residue is taken up with a little ether and treated with gaseous HCl. After cooling to 4°C overnight a precipitate of fluoxetine hydrochloride (I) with a yield of 75% is collected.

¹H NMR : 90 MHz (d₆-DMSO) 7.0-7.7 (9H), 5.75 (1H), 3.0 (2H), 2.5 (3H), 2.1-2.4 (2H).

## Claims

1. A process for the preparation of fluoxetine hydrochloride characterized in that:
a) ethyl benzoyl acetate (IX) is reduced with the aid of metal hydrides in a hydroalcoholic solvent at a controlled pH to obtain ethyl 3-hydroxy-3-phenylpropionate (X);
b) compound (X) is treated with methylamine in an alcoholic solvent to obtain the N-methylamide of 3-hydroxy-3-phenylpropionic acid (XI);
c) compound (XI) is treated with 4-trifluoromethylphenol and optionally with activating substances in an organic solvent to obtain 3-phenyl-3-[4-(trifluoromethyl)phenoxy]-N-methylpropanamide (XII)
d) compound (XII) is reduced with the aid of a metal hydride in an organic solvent to obtain fluoxetine (V), from which by treatment with HCl, fluoxetine hydrochloride is obtained.

2. A process according to claim 1, characterized in that said step a) is carried out in a solvent consisting of a mixture of methanol and water in a ratio between 4:1 and 5:1.

3. A process according to claim 1, characterized in that in said step
a) NaBH₄ in NaOH is employed in an amount between one molar equivalent and a 10% excess with respect to (IX).

4. A process according to claim 1, characterized in that said step a) is carried out at a temperature between 10 and 15°C at a pH between 6 and 7, under stirring.

5. A process according to claim 1, characterized in that said step b) is carried out in methanol, at room temperature, under stirring.

6. A process according to claim 1, characterized in that step c) is carried out in anhydrous tetrahydrofuran with stoichiometric quantities of the reagents, at a temperature between 15 and 20 °C under stirring.

7. A process according to claim 1, characterized in that as activating substances in said step c) there are employed methanesulphonic acid, 4-toluenesulfonic acid, trifluoromethanesulfonic acid or triphenyl phosphine and diethyl azodicarboxylate.

8. A process according to claim 1, characterized in that step d) is carried out by treating the said compound (XII) with LiAlH₄ in THF at the boiling point, under reflux, while stirring in a nitrogen atmosphere.

9. A process according to claim 8, characterized in that the molar ratio between LiAlH₄ and (XII) is comprised between 0.7 and 0.9.

10. A process according to claim 1, characterized in that fluoxetine hydrochloride (I) is obtained by taking up the fluoxetine (V) obtained in said step d) with ether, treating with gaseous HCl and cooling to 3-5°C.

## Patentansprüche

1. Verfahren zur Herstellung von Fluoxetin-hydrochlorid,
dadurch **gekennzeichnet,** dass
(a) Ethylbenzoylacetat (IX) mit Hilfe von Metallhydriden in einem wässrig-alkoholischen Lösungsmittel bei kontrolliertem pH reduziert wird, so dass Ethyl-3-hydroxy-3-phenylpropionat (X) erhalten wird;
(b) Verbindung (X) mit Methylamin in einem alkoholischen Lösungsmittel unter Erhalt des N-Methylamids von 3-Hydroxy-3-phenylpropionsäure (XI) behandelt wird;
(c) Verbindung (XI) mit Trifluormethylphenol und wahlweise mit aktivierenden Stoffen in einem organischen Lösungsmittel unter Erhalt von 3-Phenyl-3-[4-(trifluormethyl)phenoxy]-N-methylpropanamid (XII) behandelt wird;
(d) Verbindung (XII) mit Hilfe eines Metallhydrids in einem organischen Lösungsmittel unter Erhalt von Fluoxetin (V) reduziert wird, aus dem durch Behandlung mit HCl Fluoxetin-hydrochlorid (I) erhalten wird.

2. Verfahren gemäss Anspruch 1, dadurch **gekennzeichnet,** dass Schritt (a) in einem Lösungsmittel, bestehend aus einer Mischung aus Methanol und Wasser in einem Verhältnis zwischen 4:1 und 5:1 ausgeführt wird.

3. Verfahren gemäss Anspruch 1, dadurch **gekennzeichnet,** dass in der Stufe (a) NaBH₄ in NaOH in einer Menge zwischen 1 Moläquivalent und einem 10 %-igen Überschuss bezüglich (IX) eingesetzt wird.

4. Verfahren gemäss Anspruch 1, dadurch **gekennzeichnet,** dass die Stufe (a) bei einer Temperatur zwischen 10 und 15°C bei einem pH zwischen 6 und 7 unter Rühren ausgeführt wird.

5. Verfahren gemäss Anspruch 1, dadurch **gekennzeichnet,** dass Stufe (b) in Methanol bei Raumtemperatur unter Rühren ausgeführt wird.

6. Verfahren gemäss Anspruch 1, dadurch **gekennzeichnet,** dass Stufe (c) in wasserfreiem Tetrahydrofuran mit stöchiometrischen Reagenzienmengen bei einer Temperatur zwischen 15 und 20°C unter Rühren ausgeführt wird.

7. Verfahren gemäss Anspruch 1, dadurch **gekennzeichnet,** dass als aktivierende Stoffe in der Stufe (c) Methansulfonsäure, 4-Toluolsulfonsäure, Trifluormethansulfonsäure oder Triphenylphosphin und Diethylazodicarboxylat eingesetzt werden.

8. Verfahren gemäss Anspruch 1, dadurch **gekennzeichnet,** dass Stufe (d) durch Behandlung der Verbindung (XII) mit LiAlH₄ in THF beim Siedepunkt unter Rückfluss unter Rühren in einer Stickstoffatmosphäre ausgeführt wird.

9. Verfahren gemäss Anspruch 8, dadurch **gekennzeichnet,** dass das Molverhältnis zwischen LiAlH₄ und (XII) zwischen 0,7 und 0,9 liegt.

10. Verfahren gemäss Anspruch 1, dadurch **gekennzeichnet,** dass Fluoxetin-hydrochlorid erhalten wird, indem das Fluoxetin (V), das in der Stufe (d) erhalten wurde, mit Ether aufgenommen wird, mit gasförmigem HCl behandelt wird und auf 3 bis 5°C abgekühlt wird.

## Revendications

1. Un procédé de préparation de chlorhydrate de fluoxétine, caractérisé en ce que :
a) de l'acétate d'éthylbenzoyle (IX) est réduit à l'aide d'hydrures métalliques dans un solvant hydroalcoolique à un pH contrôlé afin d'obtenir du 3-hydroxy-3-phénylpropionate d'éthyle (X);
b) le composé (X) est traité avec de la méthylamine dans un solvant alcoolique pour obtenir du N-méthylamide de l'acide 3-hydroxy-3-phénylpropionique (XI) ;
c) le composé (XI) est traité avec du 4-trifluoromé thylphénol et éventuellement avec des substances d'activation dans un solvant organique pour obtenir du 3-phényl-3-[4-(trifluorométhyl) phenoxy]-N-méthylpropanamide (XII)
d) le composé (XII) est réduit à l'aide dun hydrure métalliques dans un solvant organique pour obtenir de la fluoxétine (V), à partir de laquelle on obtient par traitement avec du HCl, du chlorhydrate de fluoxétine.

2. Un procédé selon la revendication 1, caractérisé en ce que ladite étape a) est mise en oeuvre dans un solvant consistant en un mélange de méthanol et d'eau dans un rapport compris entre 4/1 et 5/1.

3. Un procédé selon la revendication 1, caractérisé en ce que dans ladite étape a) NaBH₄ est employé à une quantité comprise entre un équivalent molaire et un excès de 10% par rapport à (IX).

4. Un procédé selon la revendication 1, caractérisé n ce que ladite étape a) est mise en oeuvre à une température comprise en 10 et 15°C, à un pH compris entre 6 et 7, sous agitation.

5. Un procédé selon la revendication 1, caractérisé en ce que ladite étape b) est mise en oeuvre dans du méthanol à température ambiante, sous agitation.

6. Un procédé selon la revendication 1, caractérisé en ce que l'étape c) est mise en oeuvre dans du tétrahydrofurane anhydre avec des quantités stoechiométriques de réactifs, à une température comprise entre 15 et 20°C sous agitation.

7. Un procédé selon la revendication 1, caractérisé en ce que en tant que substances d'activation dans ladite étape c) on emploit: acide méthanesulfonique, acide 4-toluènesulfonique, triphénylphosphine acide ou trifluorométhanesulfonique et diethylazodicarboxylate.

8. Un procédé selon la revendication 1, caractérisé en ce que l'étape d) est mise en oeuvre par traitement dudit composé (XII) avec LiA1H₄ dans du THF au point d'ébullition, sous reflux, sous agitation sous une atmosphère d'azote.

9. Un procédé selon la revendication 8, caractérisé en ce que le rapport molaire LiAlH₄ (XII) est compris entre 0,7 et 0,9.

10. Un procédé selon la revendication 1, caractérisé en ce que le chlorhydrate de fluoxétine (I) est obtenu par reprise de la fluoxétine (V), obtenue au cours de ladite étape d), avec de l'éther, traitement avec du HCl gazeux et refroidissement à 3-5°C.
